# EUROPEAN PATENT SPECIFICATION

(11) **EP 1 370 254 B1**
(45) Date of publication and mention of the grant of the patent: **16.01.2008**
(21) Application number: 01998342.8
(22) Date of filing: 30.11.2001
(51) Int. Cl.: A61K 31/132, A61P 27/02

(54) **COPPER CHELATORS FOR TREATING OCULAR INFLAMMATION**
KUPFERCHELATBILDNER ZUR BEHANDLUNG VON AUGENENTZÜNDUNGEN
AGENTS DE CHELATION DU CUIVRE SERVANT A TRAITER L'INFLAMMATION OCULAIRE

(30) Priority: 01.12.2000 US 250164 P
(43) Date of publication of application: 17.12.2003
(73) Proprietor: Radical Vision Therapeutics Inc., Vancouver, BC V6J 3P8 (CA)
(72) Inventor: WANG, Xuefeng, Vancouver, British Columbia V6T 1N7 (CA); CUI, Jing Zhao, Vancouver, British Columbia V5Z 3J8 (CA); MATSUBARA, Joanne, A., Vancouver, British Columbia V6J 3P8 (CA)
(74) Representative: Brants, Johan P.E.
(86) International application number: PCT/CA2001/001735
(87) International publication number: WO 2002/043722

(56) References cited:
- US-A- 4 904 698
- US-A- 5 834 457
- KREJCÍ L: "[A new neutralizing agent diethylenetriamine (DETA) in the treatment of acid burns of the eyes. II. Clinical part]" CESKOSLOVENSKA OFTALMOLOGIE. CZECHOSLOVAKIA JUL 1967, vol. 23, no. 4, July 1967 (1967-07), pages 283-287, XP001149523 ISSN: 0009-059X
- KREJCN L ET AL: "DIETHYLENETRIAMINE AS FIRST AID IN EYE BURNS WITH PHENOL AND ALDEHYDES" CESKOSLOVENSKA OFTALMOLOGIE, CESKA LEKARSKA SPOLECNOST J.E. PURKYNE, PRAGUE,, CZ, vol. 24, no. 2, March 1968 (1968-03), pages 132-134, XP001149522 ISSN: 0009-059X
- MCGAHAN M C ET AL: "COPPER DEPLETION AND D PENICILLAMINE TREATMENT ALTER SOME PARAMETERS OF THE OCULAR INFLAMMATORY RESPONSE" INVESTIGATIVE OPHTHALMOLOGY & VISUAL SCIENCE, vol. 29, no. ABSTR. ISSUE, 1988, page 287 XP008015809 ISSN: 0146-0404
- MOREAU J M ET AL: "Phospholipase A(2) in rabbit tears: a host defense against Staphylococcus aureus." INVESTIGATIVE OPHTHALMOLOGY & VISUAL SCIENCE. UNITED STATES SEP 2001, vol. 42, no. 10, September 2001 (2001-09), pages 2347-2354, XP008015789 ISSN: 0146-0404
- MCGAHAN M C ET AL: "The pathophysiology of the ocular microenvironment. II. Copper-induced ocular inflammation and hypotony." EXPERIMENTAL EYE RESEARCH. ENGLAND JUN 1986, vol. 42, no. 6, June 1986 (1986-06), pages 595-605, XP008016955 ISSN: 0014-4835
- BOLOS C A ET AL: "Structure-activity relationships for some diamine, triamine and Schiff base derivatives and their copper(II) complexes." METAL-BASED DRUGS, vol. 5, no. 6, 1998, pages 323-332, XP008016927 ISSN: 0793-0291
- AL-ABDULLAH ISMAIL H ET AL: "Neogenesis of pancreatic endocrine cells in copper-deprived rat models." PANCREAS, vol. 21, no. 1, July 2000 (2000-07), pages 63-68, XP008016956 ISSN: 0885-3177
- PATENT ABSTRACTS OF JAPAN vol. 2000, no. 10, 17 November 2000 (2000-11-17) & JP 2000 204037 A (TSUMURA &CO; SAKODA SABURO), 25 July 2000 (2000-07-25)

## Description

### FIELD OF THE INVENTION

In one aspect, the present invention relates to therapeutic uses of organic compounds, including nitrogen-containing compounds such as polyamines, as specified below as ophthalmological medicaments.

### BACKGROUND OF THE INVENTION

Copper chelation therapy is most often associated with Wilson's disease, an autosomal recessive disorder of copper metabolism. In this disorder, the excretion of copper into the bile appears to be defective, and reduced hepatic incorporation of copper into ceruloplasmin occurs, leading to an accumulation of copper in plasma and body tissues. Wilson's disease often leads to hepatic and/or neurologic dysfunction, and premature osteoarthritis. Two commonly used copper chelators for the treatment of Wilson's disease are D-penicillamine (DPA) and triethylenetetramine (trientine or TRIEN).

Patients with rheumatoid arthritis show elevated levels of copper and copper-binding protein, ceruloplasmin, in serum and in synovial fluid, and copper chelation therapy has been suggested for patients with rheumatoid arthritis and other inflammatory diseases (Milanino R. et al., Copper Metabolism in the Acute Inflammatory Process and its Possible Significance for a Novel Approach to the therapy of Inflammation. Int. J. Tiss. Reac. (1985) VII(6):469-474). Complexing drugs with copper has also been suggested to improve the efficacy of anti-inflammatory medications (Sorenson J.R.J. In: Milanino R. et al. editors. Copper and zinc in inflammation. Dordrecht: Kluwer Academic Publishers (1989):69-84). However, the various effects of copper on the inflammatory response in different tissues have not yet been thoroughly elucidated (Jeremy J.Y. et al. Copper Chelators Inhibit Platelet Thromboxane A2 Synthesis and Lipoxygenase Activity, in vitro. J. Drug Dev. Clin. Pract. (1995) 7:119-126). For example, it has been suggested that copper depletion, including depletion by treatment with copper chelators, may reduce levels of ceruloplasmin and thereby exacerbate some measures of occular inflammation (McGahan M.C. et al. Effects of Copper Depletion and D-penicillamine Treatment of the Ocular Inflammatory Response. Agents and Actions (1991) 34(3):405-509).

Krejcn L 1967 ("A new neutralizing agent diethylenetriamine (DETA) in the treatment of acid burns of the eyes. II Clinical part". Ceskoslovenska Oftalmologie, vol. 23, no. 4, p. 283-287) and Krejcn L et al. 1968 ("Diethylenetriamine as first aid in eye burns with phenol and aldehydes". Ceskoslovenska Oftalmologie, vol. 24, no. 2, p. 132-134) disclose the use of diethylenetriamine (DETA) for topical treatment of eye trauma. These publications do not disclose TRIEN or TETREN, nor systemic administration of copper chelators, such as TRIEN or TETREN, for the treatment of ocular inflammation.

US 4,904,698 discloses a non-irritating liquid cleansing composition for cleansing eyelids comprising, amongst others, PEG-15 tallow polyamine and optionally disodium EDTA. US 4,904,698 does not disclose TRIEN or TETREN, nor systemic administration of copper chelators, such as TRIEN or TETREN, for the treatment of ocular inflammation.

Moreau JM et al. 2001 ("Phospholipase A(2) in rabbit tears: a host defense against Staphylococcus aureus". Invest Ophthalmol Vis Sci, vol. 42, no. 10, p. 2347-54) studied anti-staphylococcal activity in rabbit tears and the involvement of phospholipase A(2) (PLA2) therein. Moreau does not disclose TRIEN or TETREN, nor systemic administration of copper chelators, such as TRIEN or TETREN, for the treatment of ocular inflammation.

Choroidal neovascularization (CNV) due to age-related macular degeneration (AMD) is a leading cause of severe vision loss in elderly people. Therapies for CNV have included laser photocoagulation and photodynamic therapy (PDT, such as methods disclosed in U.S. Patent No. 5,171,749 issued December 15, 1992, The former uses a thermal laser to destroy capillaries, with nonselective tissue damage. The latter is a relatively new therapy employing a photosensitizer, which is activated by a non-thermal laser. The therapeutic effect of PDT is generally thought to result at least partially from the formation of reactive oxygen species (ROS) or free radicals, which are cytotoxic.

Laser therapy, however, can also generate an inflammatory response, which can result in damage of normal tissue and recurrence of neovascularization, compromising therapeutic efficacy. It has been suggested that an inflammatory response, secondary to laser treatment, may play a role in inducing pathological side effects (Schmidt-Erfurth U., et al., Photodynamic therapy with verteporfin for choroidal neovascularization caused by age-related macular degeneration: results of retreatments in a phase 1 and 2 study. Arch Ophthahnol, (1999) 117(9) :1177-87; Ciulla T.A., et al., Age-related macular degeneration: a review of experimental treatments. Surv Ophthalmol, (1998) 43(2):134-46; Jackson J.R., et al., The codependence of angiogenesis and chronic inflammation. Faseb J, (1997) 11(6):457-65; Majno G., Chronic inflammation: links with angiogenesis and wound healing. Am J Pathol, (1998) 153(4):1035-9).

### SUMMARY OF THE INVENTION

In various aspects, the invention provides medicaments for systematically treating ocular inflammation using copper chelating compounds as specified in the claims. For example, the present invention provides medicaments for treating inflammation secondary to laser therapy of CNV. Laser therapy may for example include PDT and laser photocoagulation for the treatment of CNV, as well as laser therapies used to treat other eye diseases.

The invention provides the unexpected and surprising result that systemically administrating copper chelating compounds to an animal or human patient has an ocular anti-inflammatory effect. Copper chelating compounds of the invention are triethylenetetramine (CAS Registry No. 112-24-3; synonyms: TRIEN; TETA; N,N'-bis(2-aminoethyl)-ethylenediamine; N,N'-bis(2-aminoethyl)-1,2-ethanediamine; 1,8-diamino-3,6-diazaoctane; 3,6-diazaoctane-1,8-diamine; 1,4,7,10-tetraazadecane; tecza; trien; trientine; N,N'-bis(aminoethyl)ethylenediamine; DEH 24; N,N'-bis(2-aminoethyl)ethanediamine; triethylenetetraamine; formula C₆H₁₈N₄) or tetraethylenepentamine (CAS Registry No. 112-57-2; synonyms: TETREN; 1,4,7,10,13-pentaazatridecane; N-(2-aminoethyl)-N'-(2-((2-aminoethyl)amino)ethyl)- ,2-ethanediamine; 1,11-diamino-3 ,6,9-triazaundecane; D.E.H. 26; 3,6,9-triaza-1,11-undecanediamine; 3,6,9-triazaundecamethylenediamme; 3,6,9-triazaundecane- 1,11-diamine; formula C₈H₂₃N₅).

In alternative embodiments, copper chelating therapy of the invention may be used to inhibit ocular inflammatory responses caused by trauma, exposure to UV light, chemical stimuli and toxins. Thus, the present invention also provides a medicament for treating inflammation secondary to other pathological conditions, such as trauma, UV, chemical stimuli, and toxins.

### BRIEF DESCRIPTION OF THE FIGURES

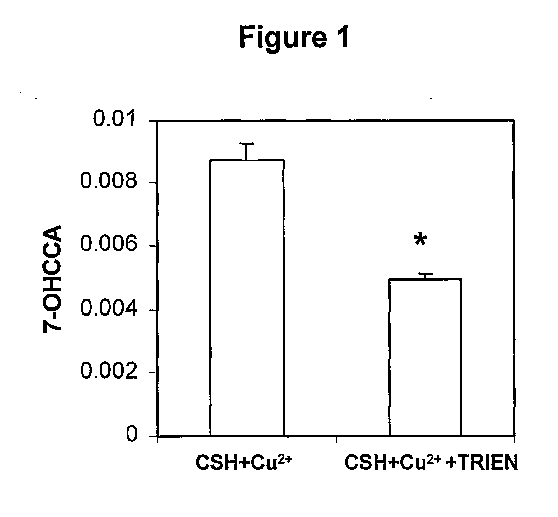

FIGURE 1 depicts the effect of copper chelator TRIEN on the generation of hydroxyl radicals (.OH) during Cu²⁺-catalyzed cysteine autoxidation. The reaction mixture included 1 mM TRIEN, 0.2 µM Cu²⁺, 100 µM cysteine, and 1 mM CCA in PBS. The control group contained all components except TRIEN. The reaction condition was pH 7.4 and 37°C in a humidified atmosphere of 100% air. Fluorescence was measured 4 hr after the reaction began. * Significantly different from the control (p < 0.01).

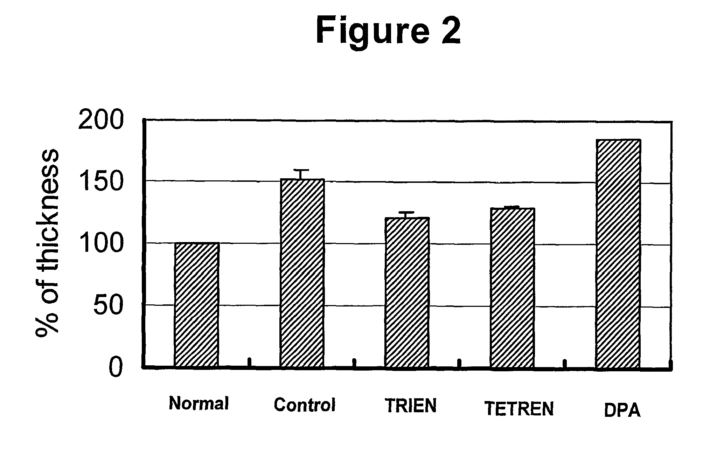

FIGURE 2 depicts the retinal thickness (edema) at day 1 post laser treatment (photocoagulation) in rats. The retinal thickness was measured from eyes of rats treated with TRIEN or TETREN or D-PA, and compared to the retinal thickness from eyes of rats injected with saline (controls) and to normal retina (no laser treatment and no drug/saline injections). Coherent Argon Dye Laser irradiation at 545 nm wavelength was delivered through a slit lamp. A total of 6 laser spots were placed separately using a setting of 50 um diameter, 0.1 sec duration and 150 mW intensity. The retinal thickness of eyes from control , animals (saline injected) is greater than that of normal (no laser treatment, no drug/saline injections) retina and eyes from animals treated with TRIEN or TETREN. The retinal thickness of eyes from animals injected with D-PA is greater than that in control eyes.

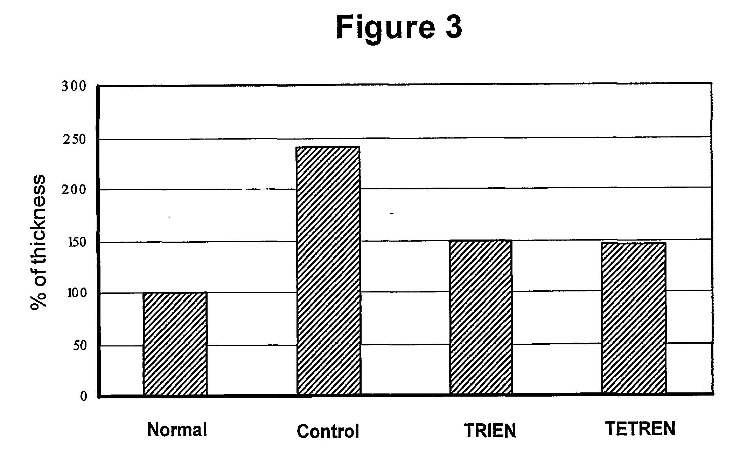

FIGURE 3 depicts the retinal thickness (edema) at day 1 post PDT in rabbits. The graph shows retinal edema at 1 day post PDT from animals treated with TRIEN or TETREN (0.2mM/day), or from control animals (saline injected). Laser light at 689 nm at a power of 600 mW/cm2 was delivered on a 5 mm spot in one eye from a diode laser (Coherent) using a slit lamp delivery system 15 minutes after verteporfin infusion. The retinal thickness from animals treated with TRIEN and TETREN is significantly less than that in control (saline injected) animals.

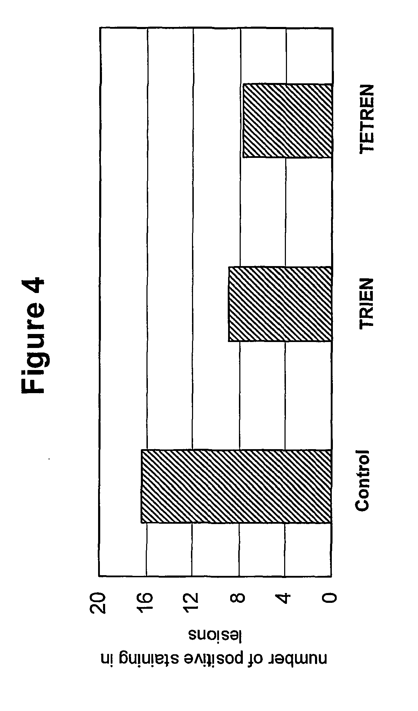

FIGURE 4 depicts the ED-1 immunostaining of retina at Day 1 post photocoagulation treatment in rats. The graph illustrates the number of ED-1 immunoreactive cells. ED-1 is a marker for macrophage cells. Coherent Argon Dye Laser irradiation at 545 nm wavelength was delivered through a slit lamp using a setting of 50 um diameter, 0.1 sec duration and 150 mW intensity. The lesions were quantified by counting the number of positive cells in an average of four 40x objective fields. The numbers of ED-1 positive cells are less in retina of TRIEN or TETREN treated animals compared to control (saline injected) animals. The numbers of ED-1 positive cells of Trientine-treated eyes were two times less than of control. The numbers of ED-1 positive cells of TETREN treated eyes were 2.5 times less than that of controls animals.

### DETAILED DESCRIPTION

In various aspects, the invention provides medicaments for systemically treating ocular inflammation using copper chelating compounds as specified in the claims. Copper chelators may for example be used to treat inflammation which is induced by laser eye therapy or other ocular injuries. In some embodiments, copper chelators may for example be used to treat an eye disease in which a symptom is ocular inflammation, such as allergic conjunctivitis, Reiter's disease, scleritis, iridocyclitis, uveitis, Vogt-Koyanagi syndrome, photophthalmia, nongranulomatous or granulomatous inflammation of uveal tract, necrosis of neoplasms, inflammation produced by foreign particles lodged in the eye, retinal light toxicity (retinal edema from light exposure).

In some embodiments, copper chelators may be used to treat patients who have undergone a laser therapy, such as laser therapy for a condition selected from the group consisting of: macular degeneration, diabetic retinopathy, proliferative diabetic retinopathy, diabetic macular edema, branch retinal vein occlusion, central serous retinopathy, vascular disorders of the fundus (angiomatosis retinae, primary retinal telangiectasis, idiopathic juxtafoveal retinal telangiectasis, acquired retinal macro aneurysms, choroidal hemangioma), retinal breaks, glaucoma (for example following laser iridotomy, argon laser trabeculoplasty, laser cyclophotocoagvlation), cataract (for example following yag laser capsulotomy), vitrectomy surgery (for example following endophotocoagulation during surgery), retinal detachment, PVR and choroidal neovascularization (for example following treatment using photocoagulation, photodynamic therapy, or transpupillary thennotherapy). In alternative embodiments, copper chelators may be administered following laser treatments such as of choroidal neovascularization using photocoagulation, photodynamic therapy, or transpupillary thermotherapy.

Copper chelators of the invention are TRIEN and TETREN.

### I. BIOLOGICAL ACTIVITY

The effectiveness of copper chelators of the invention in reducing inflammation is illustrated in various ways in the following examples of experimental procedures and Examples 1-3. Example 1 illustrates the effectiveness of the copper chelator TRIEN in inhibiting a copper catalyzed reaction. Example 2 compares the effectiveness of various copper chelators in reducing inflammation resulting from laser eye therapy. Example 3 provides evidence of immune response at the sites of inflammation.

### Experimental Procedures

### Fluorimetric assay of hydroxyl radicals

Production of OH was estimated using coumarin-3-carboxylic acid (CCA). Nonfluorescent CCA was converted by OH to highly fluorescent 7-hydroxycoumarin-3-carboxylic acid (7-OHCCA). A standard curve was calculated by measuring the fluorescence intensities of a series of concentrations of 7-OHCCA. The OH produced by cysteine auto-oxidation was represented by the corresponding 7-OHCCA concentrations.

### Administration of copper chelator

### Rabbits

The dosage of TRTEN administered was 0.2 mmol/kg.day. TRIEN injection solution was prepared as follows: 438.4mg of trientine was dissolved in 10ml distilled H₂O for a final concentration of 200 mM (or 0.2 mmol/ml). The solution was then filtered and stored it at 4°C. The pH of the solution was neutral. The injection volume was 1 ml/kg, according to the above dosage and solution concentration. TRIEN was administered intramuscularly, once a day for one week before laser treatment and 1-3 days after laser treatment. The dosage for TETREN was also 0.2 mmol/kg/day and the administration procedure was the same as for TRIEN.

### Rats

The dosage of TRIEN administered was 0.5 mmol/kg.day. TRIEN injection solution was prepared following the same method as described above for rabbits. The solution concentration was 200 mM and the injection volume was 0.25 ml/100g. TRIEN was administered intraperitoneally, once a day for one week prior to laser treatment, and for 1-3 days after laser treatment. The dosage for TETREN was also 0.5 mmol/kg/day and the administration procedure was the same as for TRIEN.

### Photodynamic therapy (PDT) in rabbits and Photocoagulations in rats

### PDT in rabbits

Six Pigmented rabbits weighing 1.5-2 kilograms were sedated for all procedures with intramuscular ketamine hydrochloride (50 mg/kg) and xylazine hydrochloride (10 mg/kg). Verteporpin for injection (2 mg/kg) was administered in a bolus by intravenous infusion.

Laser light at 689 nm at a power of 600 mW/cm2 was delivered on a 5 mm spot in one eye from a diode laser (Coherent) using a slit lamp delivery system 15 minutes after verteporfin infusion. Laser light was focused on the outer retina in the posterior pole using a contact lens. After appropriate survival periods, the animals were euthanized using intravenous pentobarbital sodium, and the eyes were enucleated and immediately placed in fixative and processed for histology 24 hours after PDT.

### Photocoagulations in Rats

Long-Evans rats weighing 400g-450g were used for all procedures with intraperitoneal injection of ketamine hydrochloride (50 mg/kg) and xylazine hydrochloride (10 mg/kg). A glass microscope cover slip was applied to the cornea using gonioscopic solution and the anaesthetized animal placed on the chin rest of a Coherent Argon Dye Laser. Dye laser irradiation at 545 nm wavelength was delivered through a slit lamp. A total of 6 laser spots were placed separately using a setting of 50 um diameter, 0.1 sec duration and 150 mW intensity. The laser spots were positioned between major retinal veins in the right eye only. The animals were sacrificed at 24 hours post photocoagulation treatment. The enucleated eyes were immediately placed in fixative and processed for histology.

### Histological Evaluation of Inflammation

The eyes were fixed with 4% paraformaldehide for 24 hours and the cornea, lens, and vitreous were removed. Eye cup photographs were taken under a microscope. The tissues were placed in freezing compound and frozen with liquid nitrogen. Cross sections (6-8 um) were cut from each specimen. The sections were stained with hematoxylin and eosin for measurement of retinal thickness and also were stained for the presence of macrophages with ED-1, a marker for macrophage cells, using immunohistochemistry techniques and examined with microscope.

### EXAMPLE 1

### Inhibition of copper-catalyzed generation of hydroxyl radicals by copper chelator TRIEN

In order to show that copper is the major catalyst in the auto-oxidation reaction and in the generation of free radicals a copper chelator was used to inhibit the generation of free radicals. The copper chelator used was TRIEN, which is used clinically for the treatment of Wilson's disease. The effect of TRIEN on the generation of OH from cysteine autoxidation was tested by measuring the production of OH with a CCA fluorescence assay. The reaction mixture included 1 mM TRIEN, 100 µM cysteine, 0.2 µM CU²⁺, 1 mM CCA, and PBS. The control contains all components except TRIEN. As shown in Figure 1, the addition of TRIEN inhibited the generation of OH from copper-catalyzed cysteine auto-oxidation.

### EXAMPLE 2

### Effect of copper chelators on the inflammatory reaction following PDT and Photocoagulations

To determine the effect of copper chelators on the inflammatory reaction after PDT and photocoagulation, retinal thickness was measured following laser therapy.

### Retinal thickness (edema) after laser therapy

A comparison was made of rat eyes following photocoagulation therapy, where the rats received either TRIEN, TETREN, D-PA or no copper chelator (control treated) prior to laser therapy. When the rat eyes were compared on the basis of retinal thickness, the eyes from control animals (saline injected) were greater than those of the TRIEN or TETREN groups as represented in Figure 2. At twenty-four hours post photocoagulation treatment, the retinal thickness of eyes from control animals was 50% greater than that in normal retina, where no laser treatment and no copper chelator was given. The retinal thickness of eyes of TRIEN injected animals was 23% greater than that in normal retina and the retinal thickness of eyes from TETREN-treated animals was 26% greater than that in normal retina. However, when the retinal thickness of eyes from D-PA treated animals was measure they were found to be 85% greater than that of normal retinal thickness and 35% greater than that in the control animals.

A comparison was also made of rabbit eyes following PDT, where the rabbits received either TRIEN, TETREN or no copper chelator (control treated) prior to laser therapy. When the rabbit eyes were compared on the basis of retinal thickness, the eyes from control treated animals(saline injected) were greater than that eyes from animals treated with TRIEN or TETREN or untreated eyes, as shown in Figure 3. Twenty-four hours after PDT treatment, the retinal thickness of eyes from control animals (saline injected) was 140% greater than that in normal retina; the retinal thickness of TRIEN treated eyes was 50% greater than that in normal retina; the retinal thickness of TETREN treated eyes was 45% greater than that in normal retina.
The results show that the copper chelators TRIEN or TETREN were effective in reducing inflammation resulting from laser eye therapy, while DPA showed the opposite effect in rats.

### EXAMPLE 3

### Immunohistochemical examinations after laser treatment

To determine if the tissue inflammation could be correlated with the immune response were stained with macrophage antibody (ED-1). TRIEN and TETREN inhibited the immunoresponse in ocular tissues following laser therapy. All experimental animals showed a similar sequence of immunohistochemical findings, which are summarized in Figure 4. At twenty-four hours after laser treatment, the macrophage staining was clearly evident in eyes from control animals (saline injected). In eyes from TRIEN and TETREN treated animals showed fewer macrophages at the laser therapy sites. The number of ED-1 positive retina cells in TRIEN treated animals were approximately half that of the controls (saline injected). And similarly the number of ED-1 positive retina cells in TETREN treated animals were more than half that of control animals.

These results indicate that the copper chelators TRIEN or TETREN were effective in reducing immune response by macrophages following laser eye therapy.

### II. PHARMACEUTICAL PREPARATIONS AND TREATMENTS

Humans, and other animals, in particular, mammals, suffering from ocular inflammation due to laser therapy or other eye injury may be treated by systematically administering to the patient an effective amount of one or more of the copper chelators of the invention or a pharmaceutically acceptable salt thereof in a pharmaceutically acceptable carrier or diluent. The active materials can be administered orally, parenterally, intravenously, intradermally, or subcutaneously.

As used herein, the term pharmaceutically acceptable salts or complexes refers to salts or complexes that retain the desired biological activity of the above-identified compounds and exhibit minimal undesired toxicological effects. Non-limiting examples of such salts are acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acid, naphthalenedisulfonic acid, and polygalacturonic acid. For example, triethylene tetramine tetrahydrochloride or trientine hydrochloride (which may for example be available as 250 mg capsules from Merck & Co. Inc. of New Jersey, U.S.A. under the trademark SYPRINE).

The active compound may be included in the pharmaceutically acceptable carrier or diluent in an amount sufficient to deliver to a patient a therapeutically effective amount without causing serious toxic effects in the patient treated. In some embodiments, a preferred dose of the active compound for all of the above-mentioned conditions is in the range from about 0.5 to 500 mg/kg, preferably 1 to 100 mg/kg per day. The effective dosage range of the pharmaceutically acceptable derivatives can be calculated based on the weight of the parent compound to be delivered. If the derivative exhibits activity in itself, the effective dosage can be estimated as above using the weight of the derivative, or by other means known to those skilled in the art. For example, trientine hydrochloride may be administered in an initial does of 500-750 mg/day for pediatric patients and 750-1250 mg/day for adults, given in divided doses two, three or four times daily. Such doses may be increased to 2000 mg/day for adults or 1500 mg/day for pediatric patients (aged 12 or under), when clinical response to an initial dose is not adequate. Oral medications may for example be taken on an empty stomach, at least one hour before meals or two hours after meals and at least one hour apart form any other drug or food.

An effective amount of a compound of the invention may include a therapeutically effective amount or a prophylactically effective amount of the compound. A "therapeutically effective amount" generally refers to an amount effective, at dosages and for periods of time necessary, to achieve the desired therapeutic result, such as reduction or reversal of ocular inflammation. A therapeutically effective amount of copper chelator may vary according to factors such as the disease state, age, sex, and weight of the individual, and the ability of the copper chelator to elicit a desired response in the individual. Dosage regimens may be adjusted to provide the optimum therapeutic response. A therapeutically effective amount is also one in which any toxic or detrimental effects of the SS ligand are outweighed by the therapeutically beneficial effects. A "prophylactically effective amount" refers to an amount effective, at dosages and for periods of time necessary, to achieve the desired prophylactic result, such as preventing or inhibiting the rate of ocular inflammation. A prophylactically effective amount can be determined as described above for the therapeutically effective amount. Copper chelators may for example be administered in a prophylactically effective amount prior to laser eye therapy, or prior to other procedures or treatments that are likely to induce ocular inflammation. Typically, since a prophylactic dose is used in subjects prior to or at an earlier stage of disease, the prophylactically effective amount will be less than the therapeutically effective amount.

The compounds of the invention may be administered in any suitable unit dosage form, including but not limited to one containing 1 to 3000 mg, preferably 5 to 500 mg of active ingredient per unit dosage form. An oral dosage of 25-250 mg may for example be convenient.

In some embodiments, the active ingredient may for example be administered to achieve peak plasma concentrations of the active compound of about 0.1 to 100 µM, or about 1-10 µM. This may be achieved, for example, by the intravenous injection of a solution or formulation of the active ingredient, optionally in saline, or an aqueous medium or administered as a bolus of the active ingredient.

The concentration of active compound in the drug composition will depend on absorption, distribution, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed use. The active ingredient may be administered at once, or may be divided into a number of smaller doses to be administered at varying intervals of time.

Solutions or suspensions used for parenteral, intradermal or subcutaneous application may include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose.

If administered intravenously, preferred carriers are physiological saline or phosphate buffered saline (PBS). The active compound can also be administered through a transdermal patch. Methods for preparing transdermal patches are known to those skilled in the art. For example, see Brown L., and Langer R., Transdermal Delivery of Drugs, Annual Review of Medicine, 39:221-229 (1988).

In another embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid.

Liposomal suspensions may also be pharmaceutically acceptable carriers. These may be prepared according to methods known to those skilled in the art, for example, as described in US Patent No. 4,522,811. For example, liposome formulations may be prepared by dissolving appropriate lipid(s) (such as stearoyl phosphatidyl ethanolamine stearoyl phosphatidyl choline, arachadoyl phosphatidy choline, and cholesterol) in an inorganic solvent that is then evaporated, leaving behind a thin film of dried lipid on the surface of the container. An aqueous solution of the active compound or its monophosphate, and/or triphosphate derivatives are then introduced into the container. The container is then swirled by hand to free the lipid aggregates, thereby forming the liposomal suspension.

Oral compositions will generally include an inert diluent or an edible carrier. They may be enclosed in gelatin capsules or compressed into tablets. For the purpose of oral therapeutic administration, the active compound can be incorporated with excipients and used in the form of tablets, troches, or capsules. Pharmaceutically compatible binding agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or other enteric agents.

In alternative embodiments, the active compound or pharmaceutically acceptable salt or derivative thereof can be administered as a component of an elixir, suspension, syrup, wafer, chewing gum or the like. A syrup may contain, in addition to the active compounds, sucrose as a sweetening agent and certain preservatives, dyes and colorings and flavors. The compounds are administered systemically, such as by intravenous infusion or injection, for treatment of the eye. In some embodiments, anti-inflammatory treatment with copper chelators may be undertaken following photodynamic therapy (such as is described in U.S. 5,798,349 issued 25 August 1998.

The active compound or pharmaceutically acceptable derivatives or salts thereof can also be administered with other active materials that do not impair the desired action, or with materials that supplement the desired action, such as antibiotics, antifungals, antiinflammatories, or antiviral compounds. The active compounds can be administered with lipid lowering agents such as probucol and nicotinic acid; platelet aggregation inhibitors such as aspirin; antithrombotic agents such as coumadin; calcium channel blockers such as varapamil, diltiazem, and nifedipine; angiotensin converting enzyme (ACE) inhibitors such as captopril and enalopril, and (3-blockers such as propanalol, terbutalol, and labetalol. The compounds can also be administered in combination with nonsteroidal antiinflammatories such as ibuprofen, indomethacin, aspirin, fenoprofen, mefenamic acid, flufenamic acid, sulindac.

Individuals being treated with copper chelators in accordance with various aspects of the invention to reduce ocular copper levels, may also limit the consumption of any supplemental dietary copper, which may have an adverse effect on the drug's performance. Numeric ranges are inclusive of the numbers defining the range. The examples herein are illustrative of various aspects of the invention.

## Claims

1. The use of triethylenetetramine (TRIEN) or tetraethylenepentamine (TETREN) to formulate a medicament for the systemic treatment of ocular inflammation.

2. The use of the copper chelator according to claim 1, wherein the copper chelator is triethylenetetramine.

3. The use of the copper chelator according to claim 1, wherein the copper chelator is tetraethylenepentamine.

4. The use of the copper chelator, according to claim 1,2 or 3, wherein the ocular inflammation results from laser eye therapy.

5. The use of the copper chelator, according to claim 1,2 or 3, wherein the ocular inflammation results from trauma.

6. The use of the copper chelator, according to claim1, 2 or 3, wherein the ocular inflammation results from exposure to ultraviolet light.

7. The use of the copper chelator, according to claim 1,2 or 3, wherein the ocular inflammation results from exposure to chemical stimuli.

8. The use of the copper chelator, according to claim 1,2 or 3, wherein the ocular inflammation results from exposure to a toxin.

9. The use of the copper chelator according to any one of claims 1 through 8, wherein the medicament is formulated for administration orally, parenterally, intravenously, intradermally or subcutaneously.

## Patentansprüche

1. Verwendung von Triethylentetramin (TRIEN) oder Tetraethylenpentamin (TETREN) zur Formulierung eines Medikaments zur systemischen Behandlung von Augenentzündung.

2. Verwendung des Kupferchelatbildners nach Anspruch 1, wobei der Kupferchelatbildner Triethylentetramin ist.

3. Verwendung des Kupferchelatbildners nach Anspruch 1, wobei der Kupferchelatbildner Tetraethylenpentamin ist.

4. Verwendung des Kupferchelatbildners nach Anspruch 1, 2 oder 3, wobei die Augenentzündung aus LaserAugentherapie resultiert.

5. Verwendung des Kupferchelatbildners nach Anspruch 1, 2 oder 3, wobei die Augenentzündung aus einem Trauma resultiert.

6. Verwendung des Kupferchelatbildners nach Anspruch 1, 2 oder 3, wobei die Augenentzündung aus Einwirkung von ultraviolettem Licht resultiert.

7. Verwendung des Kupferchelatbildners nach Anspruch 1, 2 oder 3, wobei die Augenentzündung aus Einwirkung von chemischen Stimuli resultiert.

8. Verwendung des Kupferchelatbildners nach Anspruch 1, 2 oder 3, wobei die Augenentzündung aus der Einwirkung eines Toxins resultiert.

9. Verwendung des Kupferchelatbildners nach einem der Ansprüche 1 bis 8, wobei das Medikament zur oralen, parenteralen, intravenösen, intradermalen oder subkutanen Verabreichung formuliert ist.

## Revendications

1. Utilisation de triéthylènetétramine (TRIEN) ou de tétraéthylènepentamine (TETREN) pour formuler un médicament pour le traitement systémique d'une inflammation oculaire.

2. Utilisation du chélateur du cuivre selon la revendication 1, dans laquelle le chélateur du cuivre est la triéthylènetétramine.

3. Utilisation du chélateur du cuivre selon la revendication 1, dans laquelle le chélateur du cuivre est la tétraéthylènepentamine.

4. Utilisation du chélateur du cuivre, selon la revendication 1, 2 ou 3, dans laquelle l'inflammation oculaire résulte d'un traitement au laser de l'oeil.

5. Utilisation du chélateur du cuivre, selon la revendication 1, 2 ou 3, dans laquelle l'inflammation oculaire résulte d'un traumatisme.

6. Utilisation du chélateur du cuivre, selon la revendication 1, 2 ou 3, dans laquelle l'inflammation oculaire résulte d'une exposition à de la lumière ultraviolette.

7. Utilisation du chélateur du cuivre, selon la revendication 1, 2 ou 3, dans laquelle l'inflammation oculaire résulte d'une exposition à des stimuli chimiques.

8. Utilisation du chélateur du cuivre, selon la revendication 1, 2 ou 3, dans laquelle l'inflammation oculaire résulte d'une exposition à une toxine.

9. Utilisation du chélateur du cuivre selon l'une quelconque des revendications 1 à 8, dans laquelle le médicament est formulé pour une administration orale, parentérale, intraveineuse, intradermique ou sous-cutanée.
